# FASCICULE DE BREVET EUROPEEN

(11) **EP 0 705 594 B1**
(45) Date de publication et mention de la délivrance du brevet: **18.12.1996**
(21) Numéro de dépôt: 95115559.7
(22) Date de dépôt: 02.10.1995
(51) Int. Cl.: A61K 7/043

(54) **Composition cosmétique comprenant un polymère filmogène et un composé siliconé**
Kosmetische Zusammensetzung, die ein filmbildendes Polymer und eine Siliconverbindung enthält
Cosmetic composition containing a filmforming polymer and a silicon derivative

(30) Priorité: 05.10.1994 FR 9411901
(43) Date de publication de la demande: 10.04.1996
(73) Titulaire: L'OREAL, 75008 Paris (FR)
(72) Inventeur: de la Poterie, Valérie, F-77820 Le Chatelet en Brie (FR); Mellul, Myriam, F-94240 L'Hay-les-Roses (FR)
(74) Mandataire: Dodin, Catherine

(56) Documents cités:
- EP-A- 0 214 626
- EP-A- 0 391 322
- EP-A- 0 511 092
- EP-A- 0 599 687
- EP-A- 0 627 212
- Encyclopedia of chemical technology, 3ème édition, volume 20, pages 855-856 et 922-923

## Description

La présente invention a trait à une composition cosmétique comprenant un polymère filmogène, susceptible d'être appliquée sur l'ongle, par exemple en tant que vernis ou produit de soin, ou encore en tant que base pour vernis à ongles.

Il est connu, par exemple par le document JP 60237010, une base pour vernis à ongles, constituée d'un polymère hydrosoluble en solution hydroalcoolique, ladite base étant éliminable par simple grattage, et permettant de retirer la couche de vernis par pelliculage en même temps que la couche de base, sans avoir à employer de solvants organiques.
On observe toutefois quelques inconvénients liés à ce type de produit.
Ainsi, il est généralement nécessaire de déposer au moins deux couches de base sur l'ongle, avant d'y appliquer le vernis, pour obtenir une couvrance de l'ongle suffisante permettant un pelliculage aisé. Lorsque l'on n'applique qu'une seule couche de base, le pelliculage est difficile, voire impossible, sans un grattage vigoureux de l'ongle, ce qui a pour effet d'entraîner une détérioration "mécanique" de l'état de sa surface extérieure.
D'autre part, la présence de solvants dans la sous-couche de base peut d'une part, endommager la surface extérieure de l'ongle et d'autre part, entraîner une forte adhérence de la base sur l'ongle, nécessitant alors un grattage plus important lors du pelliculage, d'où une seconde cause de risque "mécanique" de détérioration de la surface extérieure de l'ongle.
Ainsi, on peut citer la demande de brevet EP 504754 qui décrit une base pour vernis pouvant s'ôter par pelliculage, le but poursuivi dans ladite demande étant d'obtenir une base devant adhérer fortement sur la pointe de l'ongle, zone plus fragile car soumise à des sollicitations mécaniques plus importantes. La solution proposée dans cette demande est d'incorporer dans la base de la glycérine et/ou des saccharides, composés qui assurent une bonne adhérence de la base sur l'ongle.
Il est également connu par la demande EP-A-391322 d'ajouter des silicones modifiées, et notamment un copolymère diméthylpolysiloxane polyéther modifié, dans une composition de vernis à ongles comprenant une dispersion aqueuse de polyuréthane.

La demanderesse s'est posée le problème d'améliorer les compositions cosmétiques de l'art antérieur en en palliant les inconvénients et de proposer une comfacilement pelliculable et protégeant suffisamment l'ongle, tout en n'étant appliquée qu'en une seule couche.

Un objet de la présente invention est donc une composition cosmétique, susceptible d'être appliquée sur les ongles, comprenant une dispersion aqueuse de particules de polymère filmogène et un composé siliconé hydrosoluble ou dispersible dans l'eau présent à une teneur allant de 0,5 % à 10% en poids par rapport au poids total de la composition.
Un autre objet de l'invention est l'utilisation de cette composition comme base à appliquer préalablement à un vernis à ongles, base de soins pour les ongles ou vernis à ongles incolore ou coloré.
Encore un objet de l'invention est l'utilisation de cette composition pour l'obtention d'un film pelable.

Un avantage de la composition selon l'invention est d'être pelliculable facilement, sans grattage excessif, et donc de ne pas endommager la surface extérieure de l'ongle lorsqu'on ôte le vernis.
Un autre avantage est de ne pas altérer la bonne tenue du vernis appliqué ultérieurement.
Encore un autre avantage de la présente composition est de présenter une brillance et une aptitude à l'étalement sur la surface de l'ongle satisfaisantes.

La composition cosmétique selon l'invention comprend donc une dispersion aqueuse de particules de polymère filmogène.
Cette dispersion peut être préparée par l'homme du métier sur base de ses connaissances techniques générales, en particulier par dissolution du polymère, peu ou pas soluble dans l'eau, dans un solvant organique faiblement soluble dans l'eau, addition d'eau à cette solution, mélange de manière à former une émulsion, puis évaporation du solvant organique de manière à obtenir une dispersion aqueuse de polymère présentant un taux de matière sèche d'environ 30-50% en poids.

Comme polymère filmogène, on peut utiliser une dispersion aqueuse de polyuréthanne anionique, cationique, non ionique ou amphotère, de polyester-polyuréthanne, de polyéther-polyuréthanne, voire de polyurée, seule ou en mélange.
Ledit polyuréthanne peut être, par exemple, un copolymère polyuréthanne, polyurée/uréthanne ou polyurée, aliphatique, cycloaliphatique ou aromatique, comportant, seule ou en mélange,
. au moins une séquence d'origine polyester aliphatique linéaire ou ramifié et/ou cycloaliphatique et/ou aromatique, et/ou
. au moins une séquence d'origine polyéther aliphatique et/ou cycloaliphatique et/ou aromatique, et/ou
. au moins une séquence siliconée, substituée ou non, ramifiée ou non, par exemple polydiméthylsiloxane ou polyméthylphénylsiloxane, et/ou
. au moins une séquence comportant des groupes fluorés.
Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.
On peut utiliser une dispersion aqueuse de particules de polyester-polyuréthanne ou une dispersion aqueuse de particules de polyéther-polyuréthanne, seules ou en mélange dans des proportions pouvant aller de 0 à 100%.
On peut ainsi utiliser les dispersions aqueuses de polyester-polyuréthanne ou de polyéther-polyuréthanne commercialisées par exemple sous la dénomination "Sancure 2060", "Sancure 815" ou "Sancure 878" par la société Sanncor, ou "Neorez R 970" par I.C.I.
Les polyester-polyuréthanne et polyéther-polyuréthanne utilisés dans la présente invention sont de préférence de caractère anionique, ceci étant dû à la présence dans leur motif constitutif de groupements à fonction acide carboxylique ou acide sulfonique. On peut également utiliser des polyuréthannes non ioniques.

On peut également utiliser des dispersions aqueuse de polymères et/ou copolymères acryliques, acryliques styrène et vinyliques, telles que des dispersions commercialisées sous la dénomination « LR 8763 » par BASF, ou « Neocryl XK-90 » par I.C.I.. On utilise de préférence lesdites dispersions de polymères acryliques, acryliques styrène ou vinyliques en association avec une dispersion de polyuréthanne.
La teneur en matière sèche des dispersions généralement utilisées est de l'ordre de 20 à 50 % en poids et de préférence 30 à 40%, la taille des particules étant de préférence comprise entre 20 et 200 nm.
On prépare, de préférence, une composition cosmétique comprenant 80 à 97% en poids de dispersion aqueuse de polymère, soit environ 25 à 60% en poids de matière sèche de polymère dans la composition finale.

La composition cosmétique filmogène selon l'invention comprend également un composé siliconé hydrosoluble ou dispersible dans l'eau.
Ce composé peut être, par exemple, seul ou en mélange, un silicone modifié polyéther; un polydiméthylsiloxane, éventuellement oxyéthyléné comprenant éventuellement des groupements amines ou phosphates; un copolymère de silicone/protéines de blé; un sulfosuccinate de polydiméthylsiloxane.
On peut également employer comme composé siliconé selon l'invention, un agent tensioactif siliconé, soluble ou dispersible dans l'eau, qui assurera alors à la fois les fonctions de composé siliconé et d'agent tensioactif permettant d'améliorer l'aptitude à l'étalement sur les ongles de la composition cosmétique finale.
Parmi les composés siliconés remplissant également le rôle de tensioactifs, on peut citer le tensioactif actif siliconé vendu sous le nom de KF 355A par Shin Etsu. Les composés siliconés selon l'invention peuvent être anioniques, non ioniques, cationiques ou amphotères.
On choisit de préférence un composé siliconé hydrosoluble et non ionique, qui permet d'améliorer la qualité du pelliculage, c'est-à-dire d'obtenir un pelliculage plus aisé et plus stable ou plus franc.
Le composé siliconé est présent dans la composition à raison de 0,5 à 10% en poids de matière sèche, de préférence à raison de 2 à 6% en poids.

On peut ajouter à la composition selon l'invention
. un agent tensioactif usuel, tel qu'un composé fluoré par exemple (Fluorad vendu par 3M ou Forafac d'Atochem) de préférence en une quantité de 0,1à 2% en poids,
. un agent épaississant usuel, tel que la cellulose ou un dérivé cellulosique, un polymère acrylique, un dérivé de silice, une argile ou une gomme naturelle, ou tout autre type de polymère associatif usuel, de préférence en une quantité de 0,1 à 5% en poids,
. un agent mouillant usuel, de préférence en une quantité de 0 à 1% en poids,
. des charges telles que des microdispersions de cire, des copolymères fluorés pour améliorer la résistance à l'eau, des alcools et/ou des glycols pour améliorer la vitesse de séchage, de préférence en une quantité de 0 à 5% en poids.

Il est possible de colorer la composition cosmétique selon l'invention en lui ajoutant des pigments organiques, des pigments minéraux et/ou des colorants de préférence hydrosolubles, en une quantité usuelle facilement déterminable par l'homme du métier.
On peut également ajouter à cette composition des principes actifs tels que filtre UV, agent durcisseur, agent hydratant, vitamines, protéines, actif de soin des matières kératiniques, de manière à assurer la protection, le durcissement ou le soin de l'ongle. On peut ainsi obtenir une base de soins pour ongles.

La composition cosmétique selon l'invention est aisément applicable sur l'ongle en une seule couche.
On obtient ainsi une pellicule souple qui adhère bien à l'ongle et qui se pellicule facilement et d'un seul tenant.
Cette pellicule est brillante et résiste bien à l'eau, même chaude et savonneuse.
Elle recouvre l'ongle de façon satisfaisante et présente une surface extérieure lisse qui peut être recouverte d'un vernis usuel. Après avoir été recouverte avec ledit vernis, la base est toujours facilement pelliculable et permet ainsi un démaquillage aisé du vernis, en évitant l'emploi de dissolvant.

La composition cosmétique selon l'invention peut donc être utilisée en tant que base pour vernis, en tant que vernis incolore ou teinté, et/ou en tant que base de soin pour les ongles. Elle peut être également utilisée pour l'obtention d'un film pelable.

L'invention est illustrée plus en détail par les exemples suivants, donnés à titre indicatif et non limitatif.

### Exemple 1

On prépare une composition comprenant, en poids, les constituants suivants:
. 0,2% d'épaississant polyuréthanne associatif SER AD FX 1100 de Servo
. un composé siliconé en une quantité telle que définie dans le tableau ci-après,
. le complément à 100% de dispersion aqueuse de polyester-polyuréthanne à groupes carboxyliques (taux de matière sèche : 35,8 % en poids) vendue sous le nom de SANCURE 815 par Sancor.

La préparation s'effectue de la manière suivante: on ajoute l'épaississant à la dispersion aqueuse de polyuréthanne, on mélange jusqu'à obtention d'une composition homogène, puis l'on ajoute le composé siliconé.
Les résultats indiqués dans la partie « test d'arrachement » sont effectués de la manière suivante: on applique une première couche de composition sur un support en corne; on dispose sur cette première couche une bandelette en Nylon puis l'on applique une seconde couche de composition; on laisse sécher 24 h puis l'on arrache, à l'aide d'un appareil de traction, le premier film. On mesure ainsi une force d'arrachement. On effectue la même opération avec une composition témoin ne contenant pas de composé siliconé. Le résultat inscrit dans le tableau est en fait le rapport de la force d'arrachement nécessaire pour arracher le film de composition selon l'invention sur la force d'arrachement pour le film de compositiontémoin. Lorsque ce rapport est proche de 1, cela signifie que la composition testée se pellicule aussi facilement que la composition-témoin.
Les résultats indiqués dans la partie « test sur l'ongle » sont obtenus qualitativement de la manière suivante : on applique sur l'ongle une seule couche de la composition, puis un vernis solvant usuel (une ou deux couches, selon l'habitude).
On évalue le pelliculage du film de vernis en tirant, sans gratter sur le film de composition selon l'invention.
On obtient les résultats suivants :

| **Composé siliconé** | **arrachement** | **test sur l'ongle** |
|---|---|---|
| 2% de cire de silicone polyéther, non ionique (BELSIL DMC 6038 de Wacker) | 0,21 | très très facile |
| 2% de polydiméthylsiloxane oxyéthyléné à groupements amines, cationique (SILTECH AMINE 65 de Siltech) | 0,63 | très facile |
| 2% de copolymère de silicone/protéines de blé dans l'eau, non ionique (PECOSIL SW 83 de Phoenix) | 0,69 | très facile |
| 1,85% en matière active d'un mélange de silicone et glycérine, estérifié avec des acides adipiques et dodécanoïques, non ionique, à 20% de matière active (Developmental Silicone Glycerine de Siltech) | 0,40 | très facile |

La composition témoin, qui ne contient pas de composé siliconé, est difficilement pelliculable.
Le composé siliconé est à l'état dissous dans la composition finale.
Les résultats montrent que la présence d'un composé siliconé facilite nettement le pelliculage (le rapport est très inférieur à 1).

### Exemple 2

On prépare une composition comprenant, en poids, les constituants suivants:
. 0,2% d'épaississant polyuréthanne associatif SER AD FX 1100 de Servo
. 0,5% de tensioactif siliconé KF 355A de Shin Etsu (butyl-polydiméthylsiloxane oxyéthyléné oxypropyléné)
. un composé siliconé en une quantité telle que définie dans le tableau ci-après,
. le complément à 100% de dispersion aqueuse de polyester-polyuréthanne à groupes carboxyliques (taux de matière sèche : 35,8 %) vendue sous le nom de SANCURE 815 par Sancor
On obtient les résultats suivants :

| **Composé siliconé** | **arrachement** | **test sur l'ongle** |
|---|---|---|
| 2% de cire de silicone polyéther, non ionique (BELSIL DMC 6038 de Wacker) | 0,25 | très facile |
| 2% de polydiméthylsiloxane oxyéthyléné à groupements amines, cationique (SILTECH AMINE 65 de Siltech) | 1,08 | très facile |
| 2% de copolymère de silicone/protéines de blé dans l'eau, non ionique (PECOSIL SW 83 de Phoenix) | 1,08 | très facile |
| 1,85% en matière active d'un mélange de silicone et glycérine, estérifié avec des acides adipiques et dodécanoïques, non ionique, à 20% de matière active (Developmental Silicone Glycerine de Siltech) | 0,50 | très facile |
| 1,91% en matière active de sulfosuccinate de polydiméthylsiloxane sel di-sodique dans l'eau à 30% stabilisé (DMDM hydantoin 0,4%), à 30% de matière active, anionique (MACKANATE DC-30 de McIntyre | 0,75 | facile |
| 2% de polydiméthylsiloxane oxyéthyléné à groupements phosphates, anionique (PECOSIL PS-100 de Phoenix) | 0,72 | facile |
| 2% de polydiméthylsiloxane à groupements amides, non ionique (SILWAX DCA-100 de Siltech) | 0,90 | très facile |

La composition témoin contient :
. 74,5% de dispersion aqueuse de polyester-polyuréthanne à groupes carboxyliques SANCURE 815,
. 25% de SANCURE 815 contenant 0,8% d'épaississant polyuréthanne associatif SER AD FX 1100 de Servo, et
. 0,5% de tensioactif siliconé KF 355A de Shin Etsu.

Dans les compositions testées, le composé siliconé est à l'état dissous.
On constate que les compositions selon l'invention sont aussi facilement pelliculables, voir plus facilement pelliculables, que la composition-témoin.

### Exemple 3

On prépare deux compositions à appliquer sur l'ongle

### Composition A selon l'invention

. 74,5% en poids de dispersion aqueuse de polyester-polyuréthanne à groupes carboxyliques (taux de matière sèche : 35,8 %) SANCURE 815
. 25% de SANCURE 815 contenant 0,8% d'épaississant polyuréthanne associatif SER AD FX 1100 de Servo
. 0,5% de tensioactif siliconé KF 355A de Shin Etsu

### Composition B de l'art antérieur

. 0,3% d'épaississant polyuréthanne associatif SER AD FX 1100 de Servo à 10% de matière sèche
. 3,8% de glycérine
. 0,6% de silice précipitée
. 8,3% de talc, et
. 87% de dispersion aqueuse de polyester-polyuréthanne à groupes carboxyliques SANCURE 815

On applique une ou deux couches des compositions A ou B sur l'ongle, puis l'on applique une ou deux couches de vernis solvant.

On obtient les résultats suivants:

| | Composition A | Composition B |
|---|---|---|
| Une couche plus vernis | pelliculage facile | impossible de pelliculer complètement le vernis sans gratter l'ongle |
| Deux couches plus vernis | pelliculage facile | pelliculage facile mais application très longue (4 couches + séchage) |

On constate donc qu'il n'est pas possible de pelliculer un vernis lorsque l'on applique une seule couche de composition selon l'art antérieur, c'est-à-dire une composition ne contenant pas de composé siliconé.
Par contre, le pelliculage est très facile avec la composition selon l'invention.

### Exemple 4

On prépare une composition contenant, en poids, :
. 69% de dispersion aqueuse de polyester-polyuréthanne à groupes carboxyliques SANCURE 815
. 25% de SANCURE 815 contenant 0,8% d'épaississant polyuréthanne associatif SER AD FX 1100 de Servo
. 0,5% de tensioactif siliconé KF 355A de Shin Etsu
. 5% d'éthanol
. 0,5% d'un composé filtre UV
. 0,0006% d'un colorant.

On obtient une composition facilement pelliculable, qui sèche rapidement.

## Revendications

1. Composition cosmétique comprenant une dispersion aqueuse de particules de polymère filmogène et un composé siliconé hydrosoluble ou dispersables dans l'eau présent à une teneur allant de 0,5 % à 10% en poids par rapport au poids total de la composition.

2. Composition selon la revendication 1, dans laquelle le polymère filmogène est choisi parmi les polyuréthannes anioniques, cationiques, non ioniques ou amphotères, les polyester-polyuréthannes, les polyéther-polyuréthannes, les polyurées, les polymères ou copolymères acryliques, acryliques styrène, vinyliques, et leurs mélanges.

3. Composition selon l'une quelconque des revendications précédentes dans laquelle la dispersion aqueuse de polymère filmogène est présente à raison de 80-97% en poids.

4. Composition selon l'une quelconque des revendications précédentes, dans laquelle le composé siliconé est choisi parmi les silicones modifiés polyéther; les polydiméthylsiloxanes, éventuellement oxyéthylénés, comprenant éventuellement des groupements amines ou phosphates; les copolymères de silicone/protéines de blé; les sulfosuccinates de polydiméthylsiloxane; les tensioactifs siliconés, et leurs mélanges.

5. Composition selon l'une quelconque des revendications précédentes, dans laquelle le composé siliconé est hydrosoluble et non ionique.

6. Composition selon l'une quelconque des revendications précédentes, dans laquelle le composé siliconé est présent à raison de 2-6% en poids.

7. Composition selon l'une quelconque des revendications précédentes, comprenant des pigments organiques, des pigments minéraux, et/ou des colorants de préférence hydrosolubles.

8. Composition selon l'une quelconque des revendications précédentes, comprenant au moins un principe actif choisi parmi les filtres UV, les agents durcisseurs, les agents hydratants, les vitamines, les protéines et les actifs de soin des matières kératiniques.

9. Composition selon l'une quelconque des revendications précédentes se présentant sous la forme d'un vernis à ongles incolore ou coloré, d'une base pour vernis ou d'une base de soin pour ongle.

10. Utilisation de la composition selon l'une quelconque des revendications précédentes comme base à appliquer préalablement à un vernis à ongles, base de soins pour les ongles ou vernis à ongles incolore ou coloré.

11. Utilisation de la composition selon l'une quelconque des revendications 1 à 9 pour l'obtention d'un film pelable.

## Claims

1. Cosmetic composition comprising an aqueous dispersion of film-forming polymer particles and a water-soluble or water-dispersible silicone compound present at a content ranging from 0.5 % to 10 % by weight with respect to the total weight of the composition.

2. Composition according to Claim 1, in which the film-forming polymer is chosen from polyurethanes, which may be anionic, cationic, non-ionic or amphoteric, polyester-polyurethanes, polyether-polyurethanes, polyureas, acrylic, styrene acrylic or vinyl polymers or copolymers, and their mixtures.

3. Composition according to either one of the preceding claims, in which the aqueous film-forming polymer dispersion is present in the proportion of 80-97 % by weight.

4. Composition according to any one of the preceding claims, in which the silicone compound is chosen from polyether-modified silicones; optionally oxyethylenated polydimethylsiloxanes, optionally comprising amine or phosphate groups; silicone/wheat protein copolymers; polydimethylsiloxane sulphosuccinates; silicone surfactants, and their mixtures.

5. Composition according to any one of the preceding claims, in which the silicone compound is water-soluble and non ionic.

6. Composition according to any one of the preceding claims, in which the silicone compound is present in the proportion of 2-6 % by weight.

7. Composition according to any one of the preceding claims, comprising organic pigments, inorganic pigments and/or dyes which are preferably water-soluble.

8. Composition according to any one of the preceding claims, comprising at least one active principle chosen from UV screening agents, curing agents, moisturizing agents, vitamins, proteins and active principles for the care of keratinous substances.

9. Composition according to any one of the preceding claims which is provided in the form of a clear or coloured nail varnish, of a varnish base or of a nail care base.

10. Use of the composition according to any one of the preceding claims as base to be applied prior to a nail varnish, nail care base or clear or coloured nail varnish.

11. Use of the composition according to any one of Claims 1 to 9 for obtaining a peelable film.

## Patentansprüche

1. Kosmetische Zusammensetzung, die eine wäßrige Dispersion von Partikeln eines filmbildenden Polymers und eine wasserlösliche oder in Wasser dispergierbare Siliconverbindung in einem Anteil von 0,5 bis 10 Gew.-%, bezogen auf das Gesamtgewicht der Zusammensetzung, enthält.

2. Zusammensetzung nach Anspruch 1, in der das filmbildende Polymer unter anionischen, kationischen, nichtionischen und amphoteren Polyurethanen, Polyester-Polyurethanen, Polyether-Polyurethanen, Polyharnstoffen, Acrylpolymeren und Acrylcopolymeren, Acryl-Styrol-Copolymeren, Vinylpolymeren und Vinylcopolymeren sowie deren Gemischen ausgewählt ist.

3. Zusammensetzung nach einem der vorhergehenden Ansprüche, in der die wäßrige Dispersion des filmbildenden Polymers in einem Anteil von 80 bis 97 Gew.-% vorliegt.

4. Zusammensetzung nach einem der vorhergehenden Ansprüche, in der die Siliconverbindung unter polyethermodifizierten Siliconen, Polydimethylsiloxanen, die gegebenenfalls ethoxyliert sind und gegebenenfalls Amino- oder Phosphatgruppen enthalten, Silicon-WeizenproteinCopolymeren, Polydimethylsiloxansulfosuccinaten und Silicontensiden sowie deren Gemischen ausgewählt ist.

5. Zusammensetzung nach einem der vorhergehenden Ansprüche, in der die Siliconverbindung wasserlöslich und nichtionisch ist.

6. Zusammensetzung nach einem der vorhergehenden Ansprüche, in der die Siliconverbindung in einem Anteil von 2 bis 6 Gew.-% vorliegt.

7. Zusammensetzung nach einem der vorhergehenden Ansprüche, die organische Pigmente, anorganische Pigmente und/oder Färbemittel enthält, die vorzugsweise wasserlöslich sind.

8. Zusammensetzung nach einem der vorhergehenden Ansprüche, die mindestens einen Wirkstoff enthält, der unter UV-Filtern, Härtern, Hydratisierungsmitteln, Vitaminen, Proteinen und Wirkstoffen zur Pflege der Keratinmaterialien ausgewählt ist.

9. Zusammensetzung nach einem der vorhergehenden Ansprüche, die in Form eines farblosen oder farbigen Nagellacks, als Basis für Lacke oder als Basis zur Pflege der Nägel vorliegt.

10. Verwendung der Zusammensetzung nach einem der vorhergehenden Ansprüche als Basis, die vor einem Nagellack aufgetragen wird, als Basis zur Pflege der Nägel oder als farbloser oder farbiger Nagellack.

11. Verwendung der Zusammensetzung nach einem der Ansprüche 1 bis 9 zur Herstellung eines ablösbaren Films.
